# EUROPÄISCHE PATENTSCHRIFT

(11) **EP 3 354 253 B1**
(45) Veröffentlichungstag und Bekanntmachung des Hinweises auf die Patenterteilung: **03.06.2020**
(21) Anmeldenummer: 18150780.7
(22) Anmeldetag: 09.01.2018
(51) Int. Cl.: A61K 8/41, A61Q 17/04, A61K 8/49, A61K 8/35, A61K 8/37, A61K 8/29, A61K 8/39, A61K 8/73, A61K 8/44, A61K 8/34, A61K 8/67

(54) **VERWENDUNG VON DIETHYLAMINOHYDROXYBENZOYLHEXYLBENZOAT IN KOSMETISCHEN SONNENSCHUTZMITTELN**
USE OF DIETHYLAMINOHYDROXYBENZOYLHEXYLBENZOATE IN COSMETIC SUNSCREENING COMPOSITIONS
UTILISATION DE DIÉTHYLAMINO HYDROXYBENZOYL HEXYL BENZOATE DANS LES PRODUITS ANTI-SOLAIRE COSMÉTIQUES

(30) Priorität: 26.01.2017 DE 102017201235
(43) Veröffentlichungstag der Anmeldung: 01.08.2018
(73) Patentinhaber: Beiersdorf AG, 20253 Hamburg (DE)
(72) Erfinder: Schade, Tatjana, 25866 Mildstedt/Rosendahl (DE); von der Fecht, Stephanie, 22880 Wedel (DE); Schuldt, Lisa, 22547 Hamburg (DE); Sprock, Sarah, 22297 Hamburg (DE); Lerg, Heike, 22303 Hamburg (DE)

(56) Entgegenhaltungen:
- EP-A1- 2 092 930
- EP-A1- 3 093 006
- DE-A1- 10 155 963
- DE-A1-102004 009 154
- DE-A1-102007 024 346
- DE-A1-102008 021 631
- US-A1- 2014 308 220

## Beschreibung

Die vorliegende Erfindung betrifft Verfahren zur Reduzierung von Textilverfleckungen durch Zusatz von (2-[-4-(Diethylamino)-2-hydroxybenzoyl] Benzoesäurehexylester (INCI: Diethylamino Hydroxybenzoyl Hexyl Benzoate) in kosmetischen Zubereitungen sowie die Verwendung von (2-[-4-(Diethylamino)-2-hydroxybenzoyl] Benzoesäurehexylester (INCI: Diethylamino Hydroxybenzoyl Hexyl Benzoate) in kosmetischen Zubereitungen zur Reduzierung von durch die Zubereitung hervorgerufenen Textilverfleckungen.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrahd) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB-und UVA-Strahlung (Wellenlänge: 320-400 nm) führt darüber hinaus zu einer Schwächung der elastischen und kollagenen Fasern des Bindegewebes, Dies führt zu zahlreichen phototoxischen und photoallergischen Reaktionen und hat eine vorzeitige Hautalterung zur Folge.

Zum Schutz der Haut wurde daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dern Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

Kosmetische Zubereitungen wie Sonnenschutzzubereitungen, die auf die Haut aufgetragen werden, kommen regelmäßig (beabsichtigt oder unbeabsichtigt) mit Kleidungsstücken und Wäschestücken (z.B. Handtücher) in Kontakt, an denen sie (z.B. als "Abrieb" oder weil sie von den Faserstoffen "aufgesaugt" werden) zum Teil haften bleiben. Auf diese Weise entstehen, je nach Art der Inhaltsstoffe, insbesondere auf hellen Textilien Flecken und Verfärbungen. Diese Verfärbungen werden insbesondere durch nicht-wasserlösliche UVA- und Breitbandfilter hervorgerufen. Die Verfleckungen sind durch Waschen mit herkömmlichen Waschmitteln kaum zu entfernen und verstärken sich während des Waschprozesses durch Wechselwirkungen mit Ionen des Waschwassers sogar noch.

Es war daher die Aufgabe der vorliegenden Erfindung, die Nachteile des Standes der Technik zu beseitigen und eine Lösung für das Problem der Textilverfleckung in der kosmetischen Zubereitung selbst zu entwickeln und die von den Kosmetika ausgehende Textilverfleckung zu reduzieren.

Überraschend gelöst wird die Aufgabe durch ein Verfahren zur Reduzierung der durch UV-Lichtschutzfilter enthaltenden kosmetischen Zubereitungen hervorgerufenen Textilverfleckung, dadurch gekennzeichnet, dass der kosmetischen Zubereitung (2-[-4-(Diethylamino)-2-hydroxybenzoyl] Benzoesäurehexylester (INCI: Diethylamino Hydroxybenzoyl Hexyl Benzoate) zugesetzt wird.

Überraschend gelöst wird die Aufgabe durch die Verwendung von mit (2-[-4-(Diethylamino)-2-hydroxybenzoyl] Benzoesäurehexylester (INCI: Diethylamino Hydroxybenzoyl Hexyl Benzoate) in UV-Lichtschutzfilter enthaltenden kosmetischen Zubereitungen zur Reduzierung der durch die Zubereitung hervorgerufene Textilverfleckung.

Dabei geht es insbesondere darum, die durch die UV-Filter 2,4-Bis-{[4-(2-ethyl-hexyloxy)-2-hy-droxy]-phenyl}-6-(4-methoxyphenyl)-1,3,5-triazin (INCI: Bis-Ethylhexyloxyphenol methoxyphenyl Triazine) hervorgerufene Textilverfleckung zu reduzieren. Bis-Ethylhexyloxyphenol methoxyphenyl Triazine ist ein in der Kosmetik vielfach eingesetzter Breitbandfilter, der aufgrund seiner gelben Farbe und seiner generellen Schwerlöslichkeit eine Schlüsselrolle bei der Textilverfleckung spielt.

Zwar sind dem Fachmann seit mehreren Jahren kosmetische Sonnenschutzmittel mit einer UV-Filterkombination aus Bis-Ethylhexyloxyphenol methoxyphenyl Triazine und Diethylamino Hydroxybenzoyl Hexyl Benzoate bekannt. Im Stand der Technik blieb jedoch die Beobachtung bisher unentdeckt, dass Diethylamino Hydroxybenzoyl Hexyl Benzoate als reiner UV-A Filter die UV-B-Filtereigenschaften des Bis-Ethylhexyloxyphenol methoxyphenyl Triazine dergestalt beeinflusst, dass der Lichtschutzfaktor (SPF), der ein Maß des UV-B-Schutzes von Zubereitungen auf der Haut darstellt, deutlich erhöht wird.

Durch den Zusatz von Diethylamino Hydroxybenzoyl Hexyl Benzoate in Zubereitungen mit Bis-Ethylhexyloxyphenol methoxyphenyl Triazine kann dessen Einsatzkonzentration verringert werden, wodurch die durch diesen Filter hervorgerufene Textilverfleckung reduziert wird. Darüber hinaus kann durch Diethylamino Hydroxybenzoyl Hexyl Benzoate als UV-A-Filter auch die Einsatzkonzentration des UV-A-Filters 4-(tert.-Butyl)-4'-methoxydibenzoylmethan (INCI Butyl Methoxydibenzoylmethane) reduziert werden, so dass zusätzlich auch die durch diesen UV-Filter hervorgerufene, besonders schwer wieder auswaschbare Textilverfleckung reduziert wird.

Zwar kennt der Stand der Technik die US 2014/308220 A1 und die EP 3093006 A1, doch konnten diese Schriften nicht den Weg zur vorliegenden Erfindung weisen.

Die Begriffe "erfindungsgemäße Zubereitung", "erfindungsgemäß" etc. beziehen sich im Rahmen der vorliegenden Offenbarung immer auf das erfindungsgemäße Verfahren und die erfindungsgemäße Verwendung.

Es ist erfindungsgemäß vorteilhaft, wenn die Zubereitung (2-[-4-(Diethylamino)-2-hydroxybenzoyl] Benzoesäurehexylester (INCI: Diethylamino Hydroxybenzoyl Hexyl Benzoate) in einer Konzentration von 0,05 bis 1,5 Gewichts-%, bezogen auf das Gesamtgewicht der Zubereitung zugesetzt ist.

Es ist erfindungsgemäß bevorzugt, wenn die Zubereitung (2-[-4-(Diethylamino)-2-hydroxybenzoyl] Benzoesäurehexylester (INCI: Diethylamino Hydroxybenzoyl Hexyl Benzoate) in einer Konzentration von 0,05 bis 1,0 Gewichts-%, bezogen auf das Gesamtgewicht der Zubereitung zugesetzt ist.

Üblicherweise sind die erfindungsgemäßen Zubereitungen dadurch gekennzeichnet, dass die Zubereitung als weiteren UV-Filter 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol methoxyphenyl Triazine) enthält.

In einem solchen Fall ist es erfindungsgemäß vorteilhaft, wenn die Zubereitung 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol methoxyphenyl Triazine) in einer Konzentration von 0,05 bis 4,00 Gewichts-%, bezogen auf das Gesamtgewicht der Zubereitung enthält. Erfindungsgemäß bevorzugt ist eine Konzentration von 0,05 bis 2,25 Gewichts-%, bezogen auf das Gesamtgewicht der Zubereitung.

Es ist erfindungsgemäß vorteilhaft, wenn die Zubereitung als weiteren UV-Filter 4-(tert.-Butyl)-4'-methoxydibenzoylmethan (INCI Butyl Methoxydibenzoylmethane) enthält.

In einem solchen Fall ist es erfindungsgemäß vorteilhaft, wenn die Zubereitung 4-(tert.-Butyl)-4'-methoxydibenzoylmethan in einer Konzentration von 1,00 bis 5,00 Gewichts-%, bezogen auf das Gesamtgewicht der Zubereitung enthält. Erfindungsgemäß bevorzugt ist eine Konzentration von 1,25 bis 4,75 Gewichts-%, bezogen auf das Gesamtgewicht der Zubereitung.

Vorteilhafte Ausführungsformen der vorliegenden Erfindung sind dadurch gekennzeichnet, dass die Zubereitung als weiteren UV-Filter 2-Phenylbenzimidazol-5-sulfonsäure beziehungsweise deren Salze enthält.

In einem solchen Fall ist es erfindungsgemäß vorteilhaft, wenn die Zubereitung 2-Phenylbenzimidazol-5-sulfonsäure beziehungsweise deren Salze in einer Konzentration von 0,1 bis 3,00 Gewichts-%, bezogen auf das Gesamtgewicht der Zubereitung enthält. Erfindungsgemäß bevorzugt ist eine Konzentration von 0,1 bis 1,5 Gewichts-%, bezogen auf das Gesamtgewicht der Zubereitung.

Weiterhin ist es erfindungsgemäß von Vorteil, wenn die Zubereitung als weiteren UV-Filter 2-Ethylhexyl 2-hydroxybenzoat (INCI: Ethylhexyl Salicylate) und/oder 2,4,6-Tris-[anilino-(p-carbo-2'-ethyl-1'-hexyloxy)]-1,3,5-triazin (INCI: Ethylhexyl Triazone) enthält. Dabei ist eine Kombination beider UV-Filter erfindungsgemäß bevorzugt.

Enthält die erfindungsgemäße Zubereitung 2-Ethylhexyl 2-hydroxybenzoat (INCI: Ethylhexyl Salicylate), so ist es erfindungsgemäß vorteilhaft, diese Verbindung in einer Konzentration von 0,05 bis 5,00 Gewichts-%, bezogen auf das Gesamtgewicht der Zubereitung, einzusetzen.

Enthält die erfindungsgemäße Zubereitung 2,4,6-Tris-[anilino-(p-carbo-2'-ethyl-1'-hexyloxy)]-1,3,5-triazin (INCI: Ethylhexyl Triazone), so ist es erfindungsgemäß vorteilhaft, diese Verbindung in einer Konzentration von 0,1 bis 4,5 Gewichts-%, bezogen auf das Gesamtgewicht der Zubereitung, einzusetzen.

Erfindungsgemäß besonders bevorzugt ist die Zubereitung frei von 3-(4-Methylbenzyliden)-campher, 2-Hydroxy-4-methoxybenzophenon (INCI: Oxybenzon), 4-Methoxyzimtsäure(2-ethylhexyl)ester, 4-Methoxyzimtsäureisoamylester und Ethylhexyl-2-cyano-3,3-diphenylacrylat (INCI: Octocrylen).

Daraus ergibt sich als erfindungsgemäß ganz besonders bevorzugt eine Kombination aus
a) Hexyl 2-[4-(diethylamino)-2-hydroxybenzoyl]benzoate (INCI: Diethylamino hydroxybenzoyl hexyl benzoate),
b) 4-(tert.-Butyl)-4'-methoxydibenzoylmethan (INCI Butyl Methoxydibenzoylmethane)
c) Polyglyceryl-10 Stearat (INCI Polyglyceryl-10 Stearate),
d) 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol methoxyphenyl Triazine)
e) 2,4,6-Tris-[anilino-(p-carbo-2'-ethyl-1'-hexyloxy)]-1,3,5-triazin (INCI: Ethylhexyl Triazone),
f) 2-Phenylbenzimidazol-5-sulfonsäuresalze und
g) 2-Ethylhexyl 2-hydroxybenzoat (INCI: Ethylhexyl Salicylate)
wobei die Zubereitung frei ist von 3-(4-Methylbenzyliden)-campher, 2-Hydroxy-4-methoxybenzophenon (INCI: Oxybenzon), 4-Methoxyzimtsäure(2-ethylhexyl)ester, 4-Methoxyzimtsäureisoamylester und Ethylhexyl-2-cyano-3,3-diphenylacrylat (INCI: Octocrylen).

Dabei werden bevorzugt die oben angegebenen Konzentrationen eingesetzt.

Die erfindungsgemäßen Zubereitungen können als weiteren UV-Filter erfindungsgemäß vorteilhaft Titandioxid enthalten.

Enthält die erfindungsgemäße Zubereitung Titandioxid, so ist es erfindungsgemäß vorteilhaft, diese Verbindung in einer Konzentration von 0,25 bis 4,00 Gewichts-%, bezogen auf das Gesamtgewicht der Zubereitung, einzusetzen.

Es ist erfindungsgemäß vorteilhaft, wenn die erfindungsgemäße Zubereitung in Form einer Emulsion vorliegt. Dabei ist es insbesondere erfindungsgemäß bevorzugt, wenn die erfindungsgemäße Zubereitung in Form einer O/W-Emulsion vorliegt.

Erfindungsgemäß vorteilhafte Ausführungsformen der vorliegenden Erfindung sind dadurch gekennzeichnet, dass die Zubereitung Polyglycerylfettsäureester als Emulgatoren enthält.

In einem solchen Fall ist es erfindungsgemäß bevorzugt, wenn die Zubereitung als Polyglycerylfettsäureester Polyglyceryl-10 Stearat (INCI Polyglyceryl-10 Stearate) enthält.

Polyglyceryl-10 Stearat (INCI Polyglyceryl-10 Stearate) wird in diesem Falle erfindungsgemäß bevorzugt in einer Konzentration von 0,05 bis 1,25 Gewichts-%, bezogen auf das Gesamtgewicht der Zubereitung eingesetzt.

Es ist erfindungsgemäß von Vorteil, wenn die erfindungsgemäße Zubereitung eine Kombination aus Phenoxyethanol und Tocopherylacetat beziehungsweise Tocopherol enthält. Dabei ist die Kombination mit Tocopherolacetat erfindungsgemäß bevorzugt. Enthält die Zubereitung diese Stoffkombination, so ist es erfindungsgemäß vorteilhaft, Phenoxyethanol in einer Konzentration von 0,1 bis 0,9 Gewichts-%, bezogen auf das Gesamtgewicht der Zubereitung einzusetzen und Tocopherolacetat in einer Konzentration von 0,05 bis 0,5 Gewichts-%, bezogen auf das Gesamtgewicht der Zubereitung.

Erfindungsgemäß vorteilhafte Ausführungsformen der vorliegenden Erfindung sind dadurch gekennzeichnet, dass die Zubereitung Cellulose Gum und/oder Iminodisuccinat enthält. Dabei ist ein Gehalt an Cellulose Gum von 0,05 bis 0,5 Gewichts-%, bezogen auf das Gesamtgewicht der Zubereitung und ein Gehalt an Iminodisuccinat von 0,005 bis 1,00 Gewichts-%, erfindungsgemäß bevorzugt.

Erfindungsgemäß vorteilhafte Ausführungsformen der vorliegenden Erfindung sind dadurch gekennzeichnet, dass die Zubereitung einen oder mehrere der Parfümstoffe Hexylsalicylat, Linaylacetat, 2-Isobutyl-4-hydroxy-4-methyltetrahydropyran, Adipinsäurediester, Methylheptenon, alpha-Isomethylionon, Butylphenylmethylpropioal, Coumarin, Hexylcinnamal, Limonen, Linalool, Diethylsuccinat, Hydroxyisohexyl 3-Cyclohexencarboxaldehyd, Diethylsuccinat, Menthyl PCA und Citronellylmethylcrotonat, Benzyl Benzoate, AlphaIsomethylionon, Benzylalkohol, Benzylcinnamat, Benzylsalicylat, Citronellol, Eugenol, Geraniol enthält.

Erfindungsgemäß vorteilhafte Ausführungsformen sind ferner dadurch gekennzeichnet, dass die Zubereitung Ethylhexylglycerin, Propylenglycol, Butylenglycol, 2-Methylpropan-1,3-diol, 1,2-Pentandiol, 1,2-Hexandiol, 1,2-Octandiol und/oder 1,2-Decandiol enthält.

Nicht zuletzt ist es erfindungsgemäß von Vorteil, wenn die Zubereitung keine Parabene, sowie kein Methylisothiazolinon, Chlormethylisothiazolinon und DMDM-Hydantoin enthält, also frei ist von diesen Inhaltsstoffen.

Die erfindungsgemäße Zubereitung stellt üblicherweise eine Öl-in-Wasser-Emulsion (O/W-Emulsion) dar. Diese kann die für derartige Zubereitungen übliche Inhaltsstoffe enthalten.

Die erfindungsgemäße Zubereitung kann vorteilhaft Feuchthaltemittel enthalten. Als Feuchthaltemittel (Moisturizer) werden Stoffe oder Stoffgemische bezeichnet, welche kosmetischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Feuchthaltemittel (Moisturizer) im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist. Moisturizer können vorteilhaft auch als Antifaltenwirkstoffe zum Schutz vor Hautveränderungen, wie sie z. B. bei der Hautalterung auftreten, verwendet werden.

Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile® (CAS-Nr. 7631-86-9) und /oder Talkum und/oder Polyethylen, Nylon, Silica Diemthyl Silyate.

Es ist erfindungsgemäß bevorzugt, wenn die erfindungsgemäße Zubereitung Silica Dimethyl Silylate enthält.

Erfindungsgemäß vorteilhafte Ausführungsformen der vorliegenden Erfindung sind dadurch gekennzeichnet, dass die Zubereitung ein oder mehrere Öle gewählt aus der Gruppe der Verbindungen Butylene Glycol Dicaprylat/Dicaprat, Phenethyl Benzoat, C12-15 Alkyl Benzoat, Dibutyladipat; Diisopropylsebacate, Dicaprylylcarbonat, Di-C12-13 Alkyl Tartrate, Butyloctyl Salicylate, Diethylhexyl Syringylidene Malonate, Hydragenated Castor Oil Dimerate, Triheptanoin, C12-13 Alkyl Lactate, C16-17 Alkyl Benzoate, Propylheptyl Caprylate, Caprylic/Capric Triglyceride, Diethylhexyl 2,6-Naphthalate, Octyldodecanol, Caprylic/Capric Triglyceride, Ethylhexyl Cocoate, enthält.

Dabei ist es erfindungsgemäß bevorzugt, wenn die Zubereitung Dibutyladipat, Dicaprylylcarbonat und/oder C12-C15 Alkylbenzoat enthält.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol oder Polyole niedriger C-Zahl sowie deren Ether, vorzugsweise Propylenglykol, Glycerin, Elektrolyte, Selbstbräuner sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination. Weitere erfindungsgemäß vorteilhafte Verdicker sind solche mit der INCI-Bezeichnung Acrylates/C10-30 Alkyl Acrylate Crosspolymer (z. B. Pemulen TR 1, Pemulen TR 2, Carbopol 1328 von der Fa. NOVEON) sowie Aristoflex AVC (INCI: Ammonium Acryloyldimethyltaurate/VP Copolymer).

Es ist dabei erfindungsgemäß bevorzugt, wenn die Zubereitung Xanthangummi, quervernetztes Acrylat/C10-C30 Alkylacrylat Polymer und/oder Vinylpyrrolidon/Hexadecen-Copolymer enthält.

Ein Gehalt an Glycerin von mindestens 5 Gewicht-%, bezogen auf das Gesamtgewicht der Zubereitung, ist erfindungsgemäß besonders vorteilhaft.

Darüber hinaus ist es erfindungsgemäß von besonderem Vorteil, wenn die erfindungsgemäße Zubereitung Ethanol enthält.

Erfindungsgemäß vorteilhafte Ausführungsformen der vorliegenden Erfindung sind dadurch gekennzeichnet, dass die Zubereitung eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, natürliche und/oder synthetische Isoflavonoide, Flavonoide, Kreatin, Kreatinin, Taurin, ß-Alanin, Panthenol, Magnolol, Honokiol, Tocopherylacetat, Dihydroxyaceton; 8-Hexadecen-1,16-dicarbonsäure, Glycerylglycose, (2-Hydroxyethyl)harnstoff, Hyaluronsäure und/oder deren Salze und/oder Licochalcon A enthält.

Erfindungsgemäß vorteilhaft enthält die erfindungsgemäße Zubereitung Filmbildner. Filmbildner im Sinne der vorliegenden Erfindung sind Stoffe unterschiedlicher Zusammensetzung, die durch die folgende Eigenschaft charakterisiert sind; Löst man einen Filmbildner in Wasser oder anderen geeigneten Lösungsmitteln und trägt die Lösung dann auf die Haut auf, so bildet er nach dem Verdunsten des Lösemittels einen Film aus, der im wesentlichen dazu dient, die Lichtfilter auf der Haut zu fixieren und so die Wasserfestigkeit des Produktes zu steigern.

Es ist insbesondere von Vorteil, die Filmbildner aus der Gruppe der Polymere auf Basis von Polyvinylpyrrolidon (PVP) 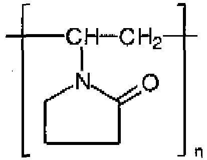 zu wählen. Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind,

Ebenfalls vorteilhaft sind weitere polymere Filmbildner, wie beispielsweise Natriumpolystryrensulfonat, welches unter der Handelsbezeichnung Flexan 130 bei der National Starch and Chemical Corp. erhältlich ist, und/oder Polyisobuten, erhältlich bei Rewo unter der Handelsbezeichnung Rewopal PIB1000. Weitere geeignete Polymere sind z.B. Polyacrylamide (Seppigel 305), Polyvinylalkohole, PVP, PVP / VA Copolymere, Polyglycole, AcrylatlOctylacralymid Copolymer (Dermacryl 79). Ebenfalls vorteilhaft ist die Verwendung von Hydriertem Rizinusöl Dimerdilinoleat (CAS 646054-62-8, INCI Hydrogenated Castor Oil Dimer Dilinoleate), das bei der Firma Kokyu Alcohol Kogyo unter dem Namen Risocast DA-H erworben werden kann oder aber auch PPG-3 Benzylethermyristat (CAS 403517-45-3), das unter dem Handelsnamen Crodamol STS bei der Firma Croda Chemicals erworben werden kann.

### Vergleichsversuch

Mit dem folgenden Versuch konnte der erfindungsgemäße Effekt beispielhaft belegt werden: Es wurde jeweils 1% des erfindungsgemäßen Hilfsstoffes zu einer Formulierung zugesetzt und die Verfleckungsreduzierende Wirkung (Reduktion b*) im Vergleich zu einer Formulierung ohne erfindungsgemäße Hilfsstoff mittels beschriebener Methode bestimmt.

Als Beleg der verbesserten Auswaschbarkeit und verminderten Fleckenbildung der erfindungsgemäßen Zubereitungen wurden in vitro Untersuchungen durchgeführt, deren Ergebnisse in der Abbildung 1 und Tabelle 1 dargestellt sind.

Es wurden verschiedene Sonnenschutzemulsionen hinsichtlich der Bildung von gelben Flecke über einen in vitro Auftragungs-/ Waschzyklus untersucht. Es wurden dabei weiße vorgewaschene Baumwollmonitore (100% Baumwolle) verwendet. Dazu wurden je 25 mg der Test-Formulierung gleichmäßig auf PMMA Schönberg Platten (5,0 x 5,0 cm) verteilt und direkt mittels Andruck auf das Testtextil übertragen. Im Anschluss wurden die verfleckten Baumwollproben für 12h unter Laborbedingungen an der Luft getrocknet.

Nach der Trocknung erfolgte eine farbmetrische Charakterisierung der entstandenen Initial-Verfleckung durch Messung des Gelbgrades mit dem Farbmessgerät spectro-color (Dr. Lange); Farbmess-Software: spectral-QC, Version Messgeometrie: d/8°, Glanzkomponente ausgeschlossen, Lichtart: D65 (entsprechend mittlerem Tageslicht), Kalibrierstandard: LZM 268, Messöffnung: 10mm, Probenhintergrund: Unterlagepapier ohne optischen Aufheller, Prüfklima: 21°C (±1°C), 41% (±4%) rel. Luftfeuchte.

Zur Auswertung wurde die Veränderung des b-Wertes aus dem CIE-Lab Farbmesssystem herangezogen. Die B-Achse charakterisiert im CIE-Lab System den Farbeindruck Gelb/ Blau, wobei positive b-Werte für eine Zunahme des Gelbanteils stehen. Je höher der b-Wert desto größer ist der Gelbeindruck.

Nach dem Messvorgang erfolgte eine separate Wäsche der Testlappen im Färbe- und Waschechtheitsgerät Linitest Plus (Atlas) (60°C, 1h, 20rpm, Ariel Compact Pulverwaschmittel,10 Metallkugeln als Beiladung) und im Anschluss ein Spülvorgang (20°C, 15min, Leitungswasser).

Nach Trocknung für 12h unter Laborbedingungen erfolgte erneut eine farbmetrische Charakterisierung der entstandenen Verfleckung durch Messung der Farbwerte wie bereits beschrieben mit dem Farbmessgerät spectro-color (Dr. Lange).

Die CIE-Lab System oder L*a*b*-Farbraum ist ein dreidimensionaler Messraum, in dem alle wahrnehmbaren Farben enthalten sind. Der Farbraum ist auf Grundlage der Gegenfarbentheorie konstruiert. Eine der wichtigsten Eigenschaften des L*a*b*-Farbmodells ist seine Geräteunabhängigkeit, das heißt, die Farben werden unabhängig von der Art ihrer Erzeugung und Wiedergabetechnik definiert.

Die entsprechende EU-Richtlinie ist DIN EN ISO 11664-4 "Farbmetrik - Teil 4: CIE 1976 L*a*b* Farbenraum". Die Koordinaten der CIELAB-Ebene werden gebildet aus dem Rot/ Grün-Wert a und dem Gelb/ Blau-Wert b. Die Helligkeitsachse L steht senkrecht auf dieser Ebene. Nach DIN 6174 sind L, a und b mit * zu schreiben, um sich gegen andere, z.B. das "Hunter-Lab"-System abzugrenzen.

| | Beispiel [% | |
|---|---|---|
| **INCI** | **1** | **2** |
| Glyceryl Stearate | 1,00 | 1,00 |
| Dibutyl Adipate | 3,00 | 3,00 |
| Aqua + Trisodium EDTA | 1,00 | 1,00 |
| Butyl Methoxydibenzoylmethane | 4,75 | 4,75 |
| Phenylbenzimidazole Sulfonic Acid | 1,00 | 1,00 |
| VP/Hexadecene Copolymer | 0,50 | 0,50 |
| Glycerin | 7,50 | 7,50 |
| Sodium Stearoyl Glutamate | 0,30 | 0,30 |
| C12-15 Alkyl Benzoate | 5,00 | 5,00 |
| Alcohol Denat. | 3,00 | 3,00 |
| Ethylhexyl Salicylate | 4,75 | 4,75 |
| Xanthan Gum | 0,40 | 0,40 |
| Silica Dimethyl Silylate | 1,00 | 1,00 |
| Stearyl Alcohol | 1,00 | 1,00 |
| Acrylates/C10-30 Alkyl Acrylate Crosspolymer | 0,10 | 0,10 |
| C18-38 Alkyl Hydroxystearoyl Stearate | 1,00 | 1,00 |
| Bis-Ethylhexyloxyphenol Methoxyphenyl Triazine | 4,00 | 4,00 |
| Butylene Glycol Dicaprylate/Dicaprate | 4,00 | 4,00 |
| Diethylamino Hydroxybenzoyl Hexyl Benzoate | | 1,00 |
| Ethylhexyl Triazone | 2,50 | 3,00 |
| Sodium Hydroxide | Add pH 7 | Add pH 7 |
| preservatives | add 100 | add 100 |
| Wasser | add 100 | add 100 |
| **Endwerte db** | **4,12** | **3,37** |

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| | | | | | | |
|---|---|---|---|---|---|---|
| **INCI** | **1** | **2** | **3** | **4** | **5** | **6** |
| Glyceryl Stearate | 1 | 1 | | | 1 | 1 |
| Sodium Stearoyl Glutamate | 0,3 | 0,3 | | | | |
| Polyglyceryl-10 Stearate | | | | | 0,8 | 0,8 |
| Glyceryl Stearate SE | | | 1 | 1 | | |
| Sodium Cetearyl Sulfate | | | 0,15 | 0,15 | | |
| Dibutyl Adipate | 3 | 4 | 3 | 4 | 3 | 4 |
| Aqua + Trisodium EDTA | 1 | 0,5 | 0,5 | 0,5 | 1 | 0,75 |
| Butyl Methoxydibenzoylmethane | 4,75 | 3 | 4,75 | 3 | 4,75 | 3 |
| Phenylbenzimidazole Sulfonic Acid | 1 | 0,5 | 1 | 0,75 | 1 | 0,5 |
| VP/Hexadecene Copolymer | 0,5 | 0,5 | 0,5 | 0,5 | 0,5 | 0,5 |
| Glycerin | 7,5 | 5 | 7,5 | 5 | 7,5 | 5 |
| C12-15 Alkyl Benzoate | 5 | 3 | 5 | 3 | 5 | 3 |
| Alcohol Denat. | 3 | 5 | 3 | 5 | 3 | 5 |
| Ethylhexyl Salicylate | 4,75 | 4,75 | 4,75 | 4,75 | 4,75 | 4,75 |
| Homosalate | | 5 | | 5 | | 5 |
| Xanthan Gum | 0,4 | 0,4 | 0,4 | 0,4 | 0,4 | 0,4 |
| Silica Dimethyl Silylate | 1 | 1 | 1 | 1 | 1 | 1 |
| Stearyl Alcohol | 1 | 0,5 | 1 | 0,5 | 1 | 0,5 |
| Acrylates/C10-30 Alkyl Acrylate Crosspolymer | 0,1 | 0,1 | 0,1 | 0,1 | 0,1 | 0,1 |
| C18-38 Alkyl Hydroxystearoyl Stearate | 1 | 0,5 | 1 | 0,5 | 1 | 0,5 |
| Bis-Ethylhexyloxyphenol Methoxyphenyl Triazine | 4 | 3 | 3 | 2 | 4 | 3 |
| Butylene Glycol Dicaprylate/Dicaprate | 4 | 3 | 4 | 3 | 4 | 3 |
| Diethylamino Hydroxybenzoyl Hexyl Benzoate | 1 | 1 | 1 | 1 | 1 | 1 |
| Ethylhexyl Triazone | 3 | 2 | 2 | 1 | 3 | 2 |
| Sodium Hydroxide | Add pH 7 | Add pH 7 | Add pH 7 | Add pH 7 | Add pH 7 | Add pH 7 |
| preservatives | add 100 | add 100 | add 100 | add 100 | add 100 | add 100 |
| Wasser | add 100 | add 100 | add 100 | add 100 | add 100 | add 100 |

## Patentansprüche

1. Verfahren zur Reduzierung der durch UV-Lichtschutzfilter enthaltenden kosmetischen Zubereitungen hervorgerufenen Textilverfleckung, **dadurch gekennzeichnet, dass** der kosmetischen Zubereitung (2-[-4-(Diethylamino)-2-hydroxybenzoyl] Benzoesäurehexylester (INCI: Diethylamino Hydroxybenzoyl Hexyl Benzoate) zugesetzt wird.

2. Verwendung von (2-[-4-(Diethylamino)-2-hydroxybenzoyl] Benzoesäurehexylester (INCI: Diethylamino Hydroxybenzoyl Hexyl Benzoate) in UV-Lichtschutzfilter enthaltenden kosmetischen Zubereitungen zur Reduzierung der durch die Zubereitung hervorgerufenen Textilverfleckung.

3. Verfahren oder Verwendung nach einem der vorhergehenden Ansprüche, **dadurch gekennzeichnet**, das der Zubereitung (2-[-4-(Diethylamino)-2-hydroxybenzoyl] Benzoesäurehexylester (INCI: Diethylamino Hydroxybenzoyl Hexyl Benzoate) in einer Konzentration von 0,05 bis 1,5 Gewichts-%, bezogen auf das Gesamtgewicht der Zubereitung zugesetzt ist.

4. Verfahren oder Verwendung nach einem der vorhergehenden Ansprüche, **dadurch gekennzeichnet, dass** die Zubereitung als weitere UV-Filter 2,4-Bis-{[4-(2-ethyl-hexyl-oxy)-2-hydroxy]-phenyl}-6-(4-methoxyphenyl)-1,3,5-triazin (INCI: Bis-Ethylhexyloxyphenol methoxyphenyl Triazine) enthält.

5. Verfahren oder Verwendung nach einem der vorhergehenden Ansprüche, **dadurch gekennzeichnet, dass** die Zubereitung als weiteren UV-Filter 4-(tert.-Butyl)-4 methoxydibenzoylmethan (INCI Butyl Methoxydibenzoylmethane) enthält.

6. Verfahren oder Verwendung nach einem der vorhergehenden Ansprüche, **dadurch gekennzeichnet, dass** die Zubereitung als weiteren UV-Filter 2-Phenylbenzimidazol-5-sulfonsäure beziehungsweise deren Salze enthält.

7. Verfahren oder Verwendung nach einem der vorhergehenden Ansprüche, **dadurch gekennzeichnet, dass** die Zubereitung als weiteren UV-Filter 2-Ethylhexyl 2-hydroxybenzoat (INCI: Ethylhexyl Salicylate) und/oder 2,4,6-Tris-[anilino-(p-carbo-2'-ethyl-1'-hexyloxy)]-1,3,5-triazin (INCI: Ethylhexyl Triazone) enthält

8. Verfahren oder Verwendung nach einem der vorhergehenden Ansprüche, **dadurch gekennzeichnet, dass** die Zubereitung als weiteren UV-FilterTitandioxid enthält.

9. Verfahren oder Verwendung nach einem der vorhergehenden Ansprüche, **dadurch gekennzeichnet, dass** die Zubereitung in Form einer Emulsion, insbesondere einer O/W-Emulsion vorliegt.

10. Verfahren oder Verwendung nach einem der vorhergehenden Ansprüche, **dadurch gekennzeichnet, dass** die Zubereitung Polyglycerylfettsäureester als Emulgatoren enthält.

11. Verfahren oder Verwendung nach einem der vorhergehenden Ansprüche, **dadurch gekennzeichnet, dass** die Zubereitung als Polyglycerylfettsäureester Polyglyceryl-10 Stearat (INCI Polyglyceryl-10 Stearate) enthält,

12. Verfahren oder Verwendung nach einem der vorhergehenden Ansprüche, **dadurch gekennzeichnet, dass** die Zubereitung eine Kombination aus Phenoxyethanol und Tocopherylacetat beziehungsweise Tocopherol enthält.

13. Verfahren oder Verwendung nach einem der vorhergehenden Ansprüche, **dadurch gekennzeichnet, dass** die Zubereitung Cellulose Gum und/oder Iminodisuccinat enthält.

## Claims

1. Method for reducing textile staining caused by cosmetic preparations comprising UV light protection filters, **characterized in that** hexyl (2-[-4-(diethylamino)-2-hydroxybenzoyl]benzoate (INCI: Diethylamino Hydroxybenzoyl Hexyl Benzoate) is added to the cosmetic preparation.

2. Use of hexyl (2-[-4-(diethylamino)-2-hydroxybenzoyl]benzoate (INCI: Diethylamino Hydroxybenzoyl Hexyl Benzoate) in cosmetic preparations comprising UV light protection filters for reducing textile staining caused by the preparation.

3. Method or use according to either of the preceding claims, **characterized in that** hexyl (2-[-4-(diethylamino)-2-hydroxybenzoyl]benzoate (INCI: Diethylamino Hydroxybenzoyl Hexyl Benzoate) is added to the preparation at a concentration of 0.05 to 1.5% by weight, based on the total weight of the preparation.

4. Method or use according to any of the preceding claims, **characterized in that** the preparation comprises 2,4-bis-{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine) as further UV filter.

5. Method or use according to any of the preceding claims, **characterized in that** the preparation comprises 4-(tert-butyl)-4'-methoxydibenzoylmethane (INCI Butyl Methoxydibenzoylmethane) as further UV filter.

6. Method or use according to any of the preceding claims, **characterized in that** the preparation comprises 2-phenylbenzimidazole-5-sulfonic acid or salts thereof as further UV filter.

7. Method or use according to any of the preceding claims, **characterized in that** the preparation comprises 2-ethylhexyl 2-hydroxybenzoate (INCI: Ethylhexyl Salicylate) and/or 2,4,6-tris[anilino-(p-carbo-2'-ethyl-1'-hexyloxy)]-1,3,5-triazine (INCI: Ethylhexyl Triazone) as further UV filter.

8. Method or use according to any of the preceding claims, **characterized in that** the preparation comprises titanium dioxide as further UV filter.

9. Method or use according to any of the preceding claims, **characterized in that** the preparation is in the form of an emulsion, especially an O/W emulsion.

10. Method or use according to any of the preceding claims, **characterized in that** the preparation comprises polyglyceryl fatty acid esters as emulsifiers.

11. Method or use according to any of the preceding claims, **characterized in that** the preparation comprises polyglyceryl-10 stearate (INCI Polyglyceryl-10 Stearate) as polyglceryl fatty acid esters.

12. Method or use according to any of the preceding claims, **characterized in that** the preparation comprises a combination of phenoxyethanol and tocopheryl acetate or tocopherol.

13. Method or use according to any of the preceding claims, **characterized in that** the preparation comprises cellulose gum and/or iminodisuccinate.

## Revendications

1. Procédé pour la réduction de la souillure de textile causée par des préparations cosmétiques contenant des filtres protecteurs anti-UV, **caractérisé en ce que** du (2-[-4-(diéthylamino)-2-hydroxybenzoyl]benzoate d'hexyle (INCI : Diethylamino Hydroxybenzoyl Hexyl Benzoate) est ajouté à la préparation cosmétique.

2. Utilisation de (2-[-4-(diéthylamino)-2-hydroxybenzoyl]benzoate d'hexyle (INCI : Diethylamino Hydroxybenzoyl Hexyl Benzoate) dans des préparations cosmétiques contenant des filtres protecteurs anti-UV pour la réduction de la souillure de textile causée par la préparation.

3. Procédé ou utilisation selon l'une quelconque des revendications précédentes, caractérisé(e) en ce que du (2-[-4-(diéthylamino)-2-hydroxybenzoyl]benzoate d'hexyle (INCI : Diethylamino Hydroxybenzoyl Hexyl Benzoate) est ajouté à la préparation en une concentration de 0,05 à 1,5 % en poids, par rapport au poids total de la préparation.

4. Procédé ou utilisation selon l'une quelconque des revendications précédentes, caractérisé(e) en ce que la préparation contient de la 2,4-bis-{[4-(2-éthyl-hexyl-oxy)-2-hydroxy]phényl}-6-(4-méthoxyphényl)-1,3,5-triazine (INCI : Bis-Ethylhexyloxyphenol methoxyphenyl Triazine) en tant que filtre UV supplémentaire.

5. Procédé ou utilisation selon l'une quelconque des revendications précédentes, caractérisé(e) en ce que la préparation contient du 4-(tert.-butyl)-4'-méthoxydibenzoylméthane (INCI Butyl Methoxydibenzoylmethane) en tant que filtre UV supplémentaire.

6. Procédé ou utilisation selon l'une quelconque des revendications précédentes, caractérisé(e) en ce que la préparation contient de l'acide 2-phénylbenzimidazole-5-sulfonique ou ses sels en tant que filtre UV supplémentaire.

7. Procédé ou utilisation selon l'une quelconque des revendications précédentes, caractérisé(e) en ce que la préparation contient du 2-hydroxybenzoate de 2-éthylhexyle (INCI : Ethylhexyl Salicylate) et/ou de la 2,4,6-tris-[anilino-(p-carbo-2'-éthyl-1 '-hexyloxy)]-1,3,5-triazine (INCI : Ethylhexyl Triazone) en tant que filtre UV supplémentaire.

8. Procédé ou utilisation selon l'une quelconque des revendications précédentes, caractérisé(e) en ce que la préparation contient du dioxyde de titane en tant que filtre UV supplémentaire.

9. Procédé ou utilisation selon l'une quelconque des revendications précédentes, caractérisé(e) en ce que la préparation se trouve sous forme d'une émulsion, en particulier d'une émulsion huile/eau.

10. Procédé ou utilisation selon l'une quelconque des revendications précédentes, caractérisé(e) en ce que la préparation contient des esters d'acides gras de polyglycéryle en tant qu'émulsifiants.

11. Procédé ou utilisation selon l'une quelconque des revendications précédentes, caractérisé(e) en ce que la préparation contient du stéarate de polyglycéryle-10 (INCI Polyglyceryl-10 Stearate) en tant qu'ester d'acide gras de polyglycéryle.

12. Procédé ou utilisation selon l'une quelconque des revendications précédentes, caractérisé(e) en ce que la préparation contient une combinaison de phénoxyéthanol et d'acétate de tocophéryle ou de tocophérol

13. Procédé ou utilisation selon l'une quelconque des revendications précédentes, caractérisé(e) en ce que la préparation contient une gomme cellulosique et/ou un iminodisuccinate.
